# EUROPÄISCHE PATENTSCHRIFT

(11) **EP 3 449 804 B1**
(45) Veröffentlichungstag und Bekanntmachung des Hinweises auf die Patenterteilung: **17.08.2022**
(21) Anmeldenummer: 17188864.7
(22) Anmeldetag: 31.08.2017
(51) Int. Cl.: A61B 1/12, A61B 90/70

(54) **VERFAHREN UND VORRICHTUNG ZUR GLEICHZEITIGEN ADAPTERANSCHLUSSÜBERPRÜFUNG UND ZUR DURCHGÄNGIGKEITSÜBERPRÜFUNG VON ENDOSKOPKANÄLEN**
METHOD AND DEVICE FOR SIMULTANEOUS VERIFICATION OF ADAPTER CONNECTION AND PERMEABILITY OF ENDOSCOPE CHANNELS
PROCÉDÉ ET DISPOSITIF DE VÉRIFICATION SIMULTANÉE DE CONNECTION D'UN ADAPTATEUR ET DE L'OUVERTURE DES CANAUX DE L'ENDOSCOPE

(43) Veröffentlichungstag der Anmeldung: 06.03.2019
(73) Patentinhaber: Cantel (Production) Germany GmbH, 88633 Heiligenberg (DE)
(72) Erfinder: Halimeh, Manaf, 86368 Gersthofen (DE); Nairz, Ewald, 86152 Augsburg (DE)
(74) Vertreter: Schaad, Balass, Menzl & Partner AG

(56) Entgegenhaltungen:
- EP-A1- 2 724 659
- EP-A2- 1 779 769
- US-A1- 2005 065 405
- US-A1- 2011 126 868
- US-A1- 2011 290 034

## Beschreibung

Die vorliegende Erfindung betrifft ein Verfahren und eine Vorrichtung zur gleichzeitigen Adapteranschlussüberprüfung und zur Durchgängigkeitsüberprüfung von Endoskopkanälen. Die konstruktionsbedingt langen und dünnen Endoskopkanäle in flexiblen Endoskopen können bei oder nach einer Endoskopanwendung verstopfen, z. B. mit geronnenem Blut oder mit Geweberesten. Ist ein Endoskopkanal verstopft, so wird er nicht durchspült und somit unzureichend aufbereitet. Ebenso muss sichergestellt werden, dass die Endoskopkanäle mit dem richtigen Adapter korrekt an die Endoskopaufbereitungsmaschine angeschlossen sind, so dass sichergestellt werden kann, dass die Reinigungs- und Desinfektionsflüssigkeit alle Endoskopkanäle erreicht.

Zum gegenwärtigen Zeitpunkt wird zur Vorbereitung des Reinigungsverfahrens erst mittels Druckluft überprüft, ob das Endoskop dicht ist, um sicherzustellen, dass die Waschflüssigkeit nicht die elektronischen Komponenten beschädigt. Nach jeder Reinigungs-, Desinfektions- und Spülstufe wird mittels einer Flüssigkeit überprüft, ob die einzelnen Endoskopkanäle korrekt an die Reinigungsapparatur angeschlossen sind sowie, dass keiner der Endoskopkanäle verstopft ist (ISO 15883-4, Kapitel 5.2.1.1).

Bei einer konventionellen Druckflussmessung ist die individuelle Ankopplung jedes einzelnen Kanales an einen Durchflussmesser, ein Druckmesser und eine Pumpe, die sich alle üblicherweise im Inneren der Reinigungsmaschine befinden, notwendig. Dies hat zur Folge, dass jedes Endoskop in der Waschkammer mit einer entsprechenden Anzahl von Anschlüssen, die heute üblicherweise zwischen fünf und zehn liegt, in der Maschine zu verbinden ist.

Zur Verbesserung der Durchströmung von Endoskopkanälen schlägt die US 5,551,462 einen Druckerhöhungsapparat vor, der an die individuellen Endoskopkanäle angeschlossen ist und dafür sorgt, dass diese unter erhöhtem Druck mit einer Reinigungsflüssigkeit durchspült werden. Eine einwandfreie Überprüfung der vollständigen Durchgängigkeit der Endoskopkanäle ist damit jedoch nicht möglich.

Zur Überprüfung des Reinigungsergebnisses von Endoskopen schlägt die DE 299 03 174 U1 vor, zusätzlich zum Endoskop einen Prüfkörper in die Maschine einzulegen und mitzureinigen, und anschliessend den Prüfkörper zu untersuchen. Ist dieser einwandfrei gereinigt, so wird davon ausgegangen, dass auch das Endoskop einwandfrei gereinigt ist. Eine solche mittelbare Prüfung erlaubt jedoch keine sicheren Rückschlüsse auf die einwandfreie Reinigung und damit Durchgängigkeit von Endoskopkanälen.

EP 2 060 276 offenbart eine Vorrichtung und ein Verfahren zum Reinigen eines Endoskops, wobei die Endoskopkanäle durch unterschiedliche Pumpleistungen gespült werden.

EP 1 779 769 offenbart ein Verfahren zum Erfassen einer ordnungsgemässen Verbindung von Anschlüssen an einem Endoskop mit Hilfe eines Endoskop-Prozessors, wobei Druckimpulse mit verschiedenen Frequenzen erzeugt werden. Die anschliessende Durchgängigkeitsüberprüfung wird mit einer Reinigungsflüssigkeit durchgeführt.

US 2005/065405 A1 offenbart eine Endoskop-Reinigungs- und Desinfektionsvorrichtung, umfassend eine Flüssigkeitszuführeinheit, eine Messeinheit und eine Erfassungseinheit, die mittels einer Vergleichsberechnung berechnet, ob die Endoskopkanäle verstopft sind.US 2011/290034 A1 offenbart ein System zur Bestimmung, ob medizinische Geräte wie Endoskope blockiert sind oder auslaufen, bevor sie einer Reinigung und/oder einem sequentiellen oder gleichzeitigen Desinfektionsprozess unterzogen werden.

EP 2724659 A1 offenbart ein Verfahren und eine Vorrichtung zur gleichzeitigen Adapteranschlussüberprüfung und Durchgängigkeitsüberprüfung von Endoskopkanälen in einer Endoskopaufbereitungsmaschine, mit einem ersten und einem zweiten Strömungsbereich, wobei ein erster Endoskopkanal des ersten Strömungsbereichs mit einem Endoskopkanal des zweiten Strömungsbereichs verbunden ist, wobei zwei Ventile alternierend Druckluft in den ersten oder in den zweiten Strömungsbereich leiten, und wobei Drücke in allen Endoskopkanälen des jeweils anderen Strömungsbereichs gemessen und ausgewertet werden.

EP 1 338 237 offenbart ein Verfahren, bei dem eine vorbestimmte Flüssigkeitsmenge mit einem vorbestimmten Druck durch die zu überprüfenden Endoskopkanäle geleitet wird und die Zeitdauer hierfür gemessen und ausgewertet wird. Die vorgegebene Flüssigkeitsmenge stammt aus einem Behälter vorbekannter Grösse, der sich auf dem Träger befindet. Sein Auslass wird auf dem Träger mit dem jeweils zu überprüfenden Endoskopkanal verbunden. Die Flüssigkeitsoberfläche wird mit Druckluft beaufschlagt, die von der Maschine stammt. Somit ist beim Beschicken der Maschine lediglich der Behälter mit einer in der Maschine befindlichen Druckluftquelle zu koppeln. Pro Endoskopkanal ist auf dem Träger vorzugsweise ein individueller Behälter vorgesehen, wobei alle Behälter über eine gemeinsame Leitung mit der Druckluftquelle verbindbar sind, so dass auch nur für die Druckluftzufuhr eine Kupplung benötigt wird. Ebenso kann zum Befüllen des Behälters mit der Flüssigkeit eine gemeinsame Leitung vorgesehen sein, die somit ebenfalls nur eine Kupplung benötigt.

Der Nachteil dieses Verfahrens ist, dass die Zykluszeiten relativ lange sind und das Gerät relativ teuer ist.

Aufgabe der vorliegenden Erfindung ist es daher, ein Verfahren und eine Vorrichtung bereitzustellen, welche eine sichere und kostengünstige Adapteranschlussüberprüfung und Durchgängigkeitsüberprüfung von Endoskopkanälen gewährleisten und kurze Zykluszeiten aufweisen.

Die Aufgabe wird durch das Verfahren gemäss Anspruch 1 und die Vorrichtung gemäss Anspruch 8 gelöst. Weitere bevorzugte Ausführungsformen sind Gegenstand der abhängigen Ansprüche.

Überraschenderweise wurde festgestellt, dass zur gleichzeitigen Adapteranschlussüberprüfung und zur Durchgängigkeitsüberprüfung von Endoskopkanälen in einer Endoskopaufbereitungsmaschine anstelle von Wasser oder einer Reinigungsflüssigkeit auch ein Gas, vorzugsweise medizinische Druckluft, verwendet werden kann, da die Druckluftmessungen ebenso präzise Resultate liefern. Das erfindungsgemässe Verfahren erfüllt den ISO Standard 15833 für Reinigungs- und Desinfektionsgeräte. Ausserdem sind die apparativen Anforderungen deutlich geringer, da allfällig austretende medizinische Druckluft weder Weichmacher herauslösen, noch das Endoskop in sonstiger Weise schädigen kann.

Das erfindungsgemässe Verfahren ist dadurch gekennzeichnet, dass die Überprüfung der Durchgängigkeit von Endoskopkanälen mit Gas, vorzugsweise mit medizinischer Druckluft erfolgt. Gleichzeitig wird die sogenannte Adapteranschlussüberprüfung durchgeführt, das heisst, es wird festgestellt, ob der richtige Adapter am Endoskop angeschlossen wurde und ob dieser Adapter korrekt an der Endoskopaufbereitungsmaschine und an den Endoskopkanälen angeschlossen wurde. Beim erfindungsgemässen Verfahren wird die Durchgängigkeitsüberprüfung und die Adapteranschlussüberprüfung in einem Prüfzyklus anhand der gleichen Messwerte durchgeführt. Das erfindungsgemässe Verfahren wird vorzugsweise für flexible Endoskope angewendet. Wenn mit dem erfindungsgemässen Verfahren festgestellt wird, dass ein oder mehrere Endoskopkanäle verstopft sind, und/oder wenn ein oder mehrere Endoskopkanäle über den Adapter nicht korrekt an die Endoskopaufbereitungsmaschine angeschlossen wurden, wird das anschliessende Reinigungsverfahren nicht gestartet. Zur Durchführung des erfindungsgemässen Verfahrens wird das Endoskop in eine Endoskopaufbereitungsmaschine gegeben und die einzelnen Endoskopkanäle mittels eines Adapters mit der erfindungsgemässen Vorrichtung fluidleitend verbunden. Wesentlich ist dabei, dass jeder Endoskopkanal, der nach dem erfindungsgemässen Verfahren mit einer Reinigungsflüssigkeit gereinigt werden muss, sowohl mit der Gasquelle, vorzugsweise der Druckluftquelle als auch mit der Flüssigkeitzufuhr fluidleitend verbunden ist. Die Druckluft wird von der Druckluftquelle über die Druckluftleitung in eine Zufuhrleitung und anschliessend über eine Verteilleitung in die Schläuche des Adapters und schliesslich in die Endoskopkanäle gepresst. Mit einer Messvorrichtung wird eine Messgrösse ermittelt, mit der festgestellt werden kann, ob ein Endoskopkanal verstopft ist, oder ob ein Endoskopkanal nicht korrekt über den Adapter mit der Endoskopaufbereitungsmaschine verbunden ist. Mögliche Messgrössen sind dabei der Kanaldruck oder die benötigte Zeitdauer, um eine bestimmte Druckluftmenge durch einen oder mehrere Endoskopkanäle zu leiten. Die gemessenen Messgrössen werden zu einer Steuerungseinheit übermittelt, mit einer Referenzgrösse abgeglichen und ausgewertet.

Vorzugsweise wird beim erfindungsgemässen Verfahren die Adapteranschlussüberprüfung und die Durchgängigkeitsüberprüfung der Endoskopkanäle gleichzeitig zu der Überprüfung der Dichtigkeit des Endoskops durchgeführt. Vorzugsweise ist der Startpunkt der beiden Verfahren im Wesentlichen gleich. Bei der Überprüfung der Dichtigkeit des Endoskops, die ebenfalls mit Gas, vorzugsweise mit Druckluft durchgeführt wird, wird sichergestellt, dass keine Lecks vorhanden sind, so dass bei der nachfolgenden Reinigung die elektronischen Komponenten nicht beschädigt werden können. Die Dichtigkeit des Endoskops wird unabhängig von der Durchgängigkeit der Endoskopkanäle über andere Anschlüsse durchgeführt. Entsprechende Verfahren sind dem Fachmann bekannt. Da das Endoskop während der Durchgängigkeitsprüfung noch keinen Kontakt mit einer Flüssigkeit hat, ist eine gleichzeitige Überprüfung möglich. Dies führt zu einer erheblichen Zyklusverkürzung, wie dem Schema 1 entnommen werden kann.

Da die Durchgängigkeitsüberprüfung der Endoskopkanäle und die Adapteranschlussüberprüfung mit Gas, vorzugsweise mit medizinischer Druckluft durchgeführt wird, kann während der Adapteranschlussüberprüfung und der Durchgängigkeitsüberprüfung die Reinigungsmaschine bereits mit der Reinigungsflüssigkeit befüllt werden, da die Druckluft immer unmittelbar zur Verfügung steht, während bei der konventionellen Überprüfung immer erst die Waschflüssigkeit in die Endoskopaufbereitungsmaschine gefüllt werden muss. Es muss einzig abgewartet werden, bis die Dichtigkeitsüberprüfung stattgefunden hat, um sicherzustellen, dass das die elektronischen Komponenten des Endoskops nicht mit der Reinigungsflüssigkeit in Berührung kommen.

Beim erfindungsgemässen Verfahren ist die Druckluft über ein Wegventil, vorzugsweise ein pneumatisches Wegventil, in mehrere Strömungsrichtungen lenkbar. So wird die individuelle Ansteuerung von einzelnen oder mehreren Strömungsbereichen ermöglicht. In einem Strömungsbereich können ein oder mehrere Adapteranschlüsse angeordnet sein, die dazu bestimmt sind, ein oder mehrere Endoskopkanäle über einen Adapter anzuschliessen. Vorzugsweise sind in einem Strömungsbereich 1 bis 4, besonders bevorzugt 3 Adapteranschlüsse angeordnet.

Vorzugsweise ist ein solches pneumatisches Wegventil ein pneumatisches Dreiwegventil. Je nach Stellung des Dreiwegventils wird der Druckluftzulaufstrom
- vollständig in einen ersten Strömungsbereich A oder
- vollständig in einen zweiten Strömungsbereich B gelenkt oder
- zwischen dem ersten Strömungsbereich A und dem zweiten Strömungsbereich B aufgeteilt.

Durch die individuelle Ansteuerung eines einzelnen Strömungsbereichs kann sichergestellt werden, dass auch Endoskopkanäle mit einem sehr kleinen Durchmesser zuverlässig mit der Druckluft durchströmt werden. Es ist beispielsweise möglich, den einzelnen Strömungsbereichen jeweils Endoskopkanäle mit ähnlichen Kanaldrücken zuzuordnen. Dadurch kann die Messgenauigkeit erhöht werden.

Unter dem Ausdruck Kanaldruck wird in der vorliegenden Erfindung derjenige Druck verstanden, der vom Drucksensor strömungsaufwärts vor der Verbindung zum Endoskopkanal gemessen wird. Unter dem Ausdruck strömungsaufwärts wird Richtung Druckluftquelle und unter strömungsabwärts Richtung Adapteranschluss und damit Richtung Endoskopkanal gemeint. Der Kanaldruck ist vom Endoskoptyp und dem spezifischen Endoskopkanal abhängig. Des Weiteren sind Endoskopkanäle zumindest teilweise miteinander verbunden, was den Kanaldruck natürlich beeinflusst.

In einer nicht-erfindungsgemäßen Ausführungsform wird der Kanaldruck von jedem einzelnen Endoskopkanal mittels eines Drucksensors gemessen. Der vom Drucksensor gemessene Kanaldruck wird einer Steuerungseinheit übermittelt und dort mit einem Referenzdruck für diesen Endoskopkanal verglichen. Entsprechende Referenzdrücke sind für jeden Endoskoptyp und dessen Endoskopkanäle in der Steuereinheit hinterlegt. Jeder Referenzdruck ist mit einer tolerierbaren Abweichung nach oben und nach unten versehen, was zu einem Referenzdruck-Toleranzbereich führt. Wenn die Druckdifferenz zwischen dem Kanaldruck und dem Referenzdruck grösser ist als der Referenzdruck-Toleranzbereich, ist der Endoskopkanal verstopft. Ist die Druckdifferenz tiefer als der Referenzdruck-Toleranzbereich, ist der Endoskopkanal nicht korrekt mit der Endoskopaufbereitungsmaschine verbunden. Mögliche Beispiele sind die Verwendung eines falschen Adapters oder das nicht dichte Anschliessen des richtigen Adapters. Wenn festgestellt wird, dass der Endoskopkanal verstopft ist und/oder der Endoskopkanal nicht korrekt mit der Endoskopaufbereitungsmaschine verbunden ist, wird das Reinigungsverfahren nicht gestartet. Vorzugsweise wird der Anwender durch ein optisches oder akustisches Signal darauf aufmerksam gemacht. Diese Ausführungsform erfordert die gleiche Anzahl von Drucksensoren wie Endoskopkanäle vorhanden sind, auf der anderen Seite liefert dieses Verfahren auch ausgesprochen zuverlässige Resultate und erlaubt ein exaktes Identifizieren der Störungsquelle. Bei dieser Ausführungsform können alle Kanaldrücke gleichzeitig gemessen oder es können einzelne Strömungsbereiche angesteuert werden. In einem ersten Schritt wird nur der erste Strömungsbereich A angesteuert, und die Druckluft wird durch n an den ersten Strömungsbereich A angeschlossene Endoskopkanäle gepresst. Dabei werden die Endoskopdrücke mittels eines Drucksensors von sämtlichen Endoskopkanälen gemessen und der Steuereinheit übermittelt. Das heisst, sowohl von den Endoskopkanälen, die an den ersten Strömungsbereich A angeschlossen sind als auch von denen, die an den zweiten Strömungsbereich B angeschlossen sind, wird der Kanaldruck gemessen.

In einem zweiten Schritt wird nur der zweite Strömungsbereich B angesteuert und die Druckluft durch m an den zweiten Strömungsbereich B angeschlossene Endoskopkanäle gepresst. Wieder werden die Endoskopdrücke von sämtlichen Endoskopkanälen mittels eines Drucksensors gemessen und der Steuereinheit übermittelt.

In einem dritten Schritt werden beide Strömungsbereiche A und B angesteuert und die Druckluft durch sämtliche angeschlossene Endoskopkanäle gepresst und die Endoskopdrücke von sämtlichen Endoskopkanälen mittels eines Drucksensors gemessen und der Steuereinheit übermittelt. Der dritte Schritt ist optional und dient nur einer zusätzlichen Kontrolle.

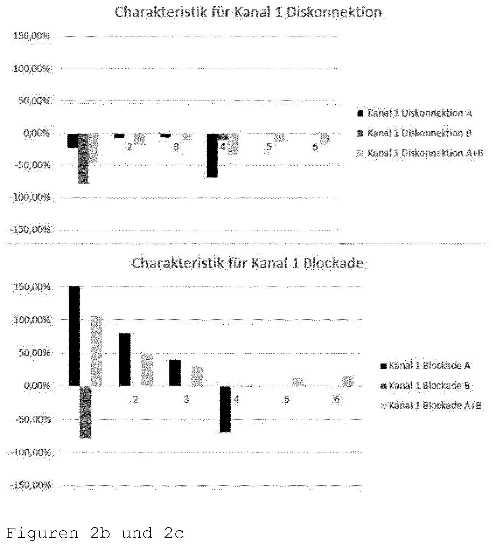

Durch dieses Vorgehen erhält man insbesondere bei Endoskopkanälen, die miteinander verbunden sind, aber mit unterschiedlichen Strömungsbereichen verbunden sind, wertvolle Zusatzinformationen, da sich ein nicht verbundener Endoskopkanal oder ein blockierter Endoskopkanal im gemessenen Druck des damit verbundenen Endoskopkanals zeigen kann (siehe Figuren 2b und 2c).

In der erfindungsgemäßen Ausführungsform sind n Endoskopkanäle an einem ersten Strömungsbereich A und m Endoskopkanäle an einem zweiten Strömungsbereich B angeschlossen, wobei ein erster Endoskopkanal, der an den Strömungsbereich A angeschlossen ist, mit einem an dem Strömungsbereich B angeschlossenen Endoskopkanal verbunden ist. Dabei wird in einem ersten Schritt nur der erste Strömungsbereich A angesteuert und die Druckluft wird durch n an den ersten Strömungsbereich A angeschlossene Endoskopkanäle gepresst. In diesem ersten Strömungsbereich A wird bei einem ersten Endoskopkanal der Kanaldruck nicht gemessen und bei allen anderen, das heisst bei n-1 + m Endoskopkanälen wird der Kanaldruck von jedem einzelnen Endoskopkanal mittels eines Drucksensors gemessen und der Steuerungseinheit übermittelt. Dort wird jeder Kanaldruck mit dem Referenzdruck für diesen Endoskopkanal verglichen und ausgewertet.

In einem zweiten Schritt wird nur der zweite Strömungsbereich B angesteuert und die Druckluft durch m an den zweiten Strömungsbereich B angeschlossene Endoskopkanäle gepresst. In diesem zweiten Strömungsbereich B werden sämtliche Kanaldrücke von allen m + n-1 Endoskopkanälen mittels eines Drucksensors gemessen und der Steuerungseinheit übermittelt. Dort wird jeder gemessene Kanaldruck mit dem Referenzdruck für diesen Endoskopkanal verglichen und ausgewertet.

In einem dritten Schritt werden beide Strömungsbereiche A und B angesteuert und die Druckluft durch sämtliche angeschlossene Endoskopkanäle gepresst und für die n-1 und für die m Endoskopkanäle jeweils der Kanaldruck mittels eines Drucksensors gemessen und der Steuereinheit übermittelt. Der dritte Schritt ist nur optional und dient einer zusätzlichen Kontrolle. Sämtliche gemessenen Kanaldrücke werden in der Steuereinheit mit den Referenzdrücken verglichen. Dadurch, dass der erste Endoskopkanal mit einem weiteren Endoskopkanal verbunden ist, kann trotzdem festgestellt werden, ob der erste Endoskopkanal verstopft ist oder nicht. Durch diese Ausführungsform kann die Vorrichtung kostengünstiger gestaltet werden.

In einer weiteren Ausführungsform des erfindungsgemässen Verfahrens wird eine vorbestimmte Druckluftmenge durch die zu überprüfenden Endoskopkanäle geleitet, die Zeitdauer hierfür gemessen und ausgewertet. Dazu wird in einem ersten Schritt ein Behälter mit einem vorzugsweise integrierten Luftdrucksensor mit Druckluft gefüllt und der Zustand «Behälter voll» wird von dem Drucksensor erfasst und der Steuereinheit gemeldet. In einem zweiten Schritt wird die Druckluft ab einem von der Steuereinheit registrierten Zeitpunkt to durch die angeschlossene Endoskopkanäle gepresst. Ist alle Druckluft aus dem Behälter durch die Endoskopkanäle gepresst, so wird der Zustand "Behälter leer" von dem Luftdrucksensor erfasst, an die Steuereinheit gemeldet und der Zeitpunkt t₁ erfasst. Die Steuereinheit ermittelt dann die Zeitdauer t₁-t₀, die benötigt wurde, um sämtliche Druckluft aus dem Behälter durch die Endoskopkanäle zu pressen. Bei freien Endoskopkanälen, bekanntem Querschnitt der Endoskopkanäle, bekanntem Volumen des Behälters und bekanntem, konstantem Druck der Druckluft muss der Behälter innerhalb einer vorgegebenen Referenzdauer vollständig entleert sein. Die ermittelte Zeitdauer t₁-t₀ wird in der Steuereinheit mit der Referenzdauer verglichen und ausgewertet. Weicht die ermittelte Zeitdauer von der Referenzdauer ab, ist ein Endoskopkanal blockiert und/oder nicht korrekt angeschlossen und das Verfahren wird abgebrochen.

Die vorliegende Erfindung richtet sich auch auf eine Vorrichtung zur Durchführung des erfindungsgemässen Verfahrens. Eine solche Vorrichtung enthält die Merkmale gemäß des Anspruchs 8.

Die Messwerte werden erfindungsgemäss aufgrund des Druckluftstroms ermittelt. Für die Verbindung zwischen der erfindungsgemässen Vorrichtung und den Endoskopkanälen wird ein Adapter verwendet. Der Adapter kann beispielsweise aus einfachen Schläuchen mit entsprechenden Kupplungsstücken an den Enden bestehen, die jeweils an einem Ende an einen Anschluss der Endoskopaufbereitungsmaschine passen und am anderen Ende an eine anzuschliessende Öffnung eines Endoskops. Die Messeinrichtungen können einzeln oder über eine Sammelleitung mit der Steuerungseinheit verbunden sein. Auch eine drahtlose Verbindung ist möglich.

Erfindungsgemäß ist dabei die Messeinrichtung ein Drucksensor. Hierfür können mechanische, optische, elektrische oder sonstige Sensoren verwendet werden, die in an sich bekannter Weise einen Druck messen.

Um die Zykluszeit zu verkürzen und um sicherzustellen, dass sämtliche Endoskopkanäle von der Druckluft erreicht werden, kann der Druckluftstrom in verschiedene Strömungsbereiche gelenkt oder zwischen verschiedenen Strömungsbereichen aufgeteilt werden. Dies kann beispielsweise über mehrere Ventile, wie beispielsweise hydraulische Ventile, geschehen. Erfindungsgemäß wird jedoch der Druckluftstrom über ein Wegventil , besonders bevorzugt ein pneumatisches Wegventil, gesteuert, da dieses praktischer und kostengünstiger ist. Besonders bevorzugt ist dabei ein Dreiwegventil, mit dem ein erster und/oder ein zweiter Strömungsbereich angesteuert werden kann.

Nicht gemäß der Erfindung ist auf jeder Verteilleitung für jeden Endoskopkanal ein Drucksensor angeordnet. In der erfindungsgemäßen Ausführungsform weist im ersten Strömungsbereich A eine einzige Verteilleitung keinen Drucksensor auf. Diese Verteilleitung ist dazu bestimmt, an einen Endoskopkanal angeschlossen zu werden, der mit einem weiteren Endoskopkanal verbunden ist, der dazu bestimmt ist, an eine Verteilleitung eines zweiten Strömungsbereich B angeschlossen zu werden. Die übrigen Verteilleitungen weisen alle einen Drucksensor aus. Ausserdem kann im zweiten Strömungsbereich B eine einzige Verteilleitung kein Rückschlagventil aufweisen. Die übrigen Verteilleitungen weisen alle ein Rückschlagventil auf. Es sind auch Ausführungsformen möglich, bei denen nur der Drucksensor in einer Verteilleitung oder nur das Rückschlagventil in einer Verteilleitung fehlt. Solche Ausführungsformen sind kostengünstiger als diejenigen, in denen für jeden Endoskopkanal ein Drucksensor angeordnet ist, allerdings kann mit Hilfe dieser Ausführungsformen, wie oben detailliert festgehalten, mit guter Sicherheit festgestellt werden, ob ein Endoskopkanal blockiert ist oder ob ein Endoskopkanal nicht korrekt mit der erfindungsgemässen Vorrichtung verbunden ist.

Eine weitere Möglichkeit sicherzustellen, dass sämtliche Endoskopkanäle mit genügend Druckluft beaufschlagt werden, ist, dass Düsen mit einem grösseren Einlassdurchmesser und einem kleineren Auslassdurchmesser die Druckluft kanalisieren können. Alternativ können auch kostengünstigere, in einer Platte angeordnete Löcher verwendet werden, wobei für je eine Verteilleitung ein Loch in der Platte angeordnet ist. Die Löcher übernehmen dabei die Düsenfunktion.

Vorzugsweise ist strömungsaufwärts vor jedem Drucksensor ein Rückschlagventil angeordnet, um sicherzustellen, dass die Druckluft eines allfällig blockierten Endoskopkanals nicht zurückströmen und um ein Übersprechen der Endoskopkanäle zu verhindern. Dies hat ausserdem den Vorteil, dass auch Kanaldrücke von Endoskopkanälen gemessen werden können, die an einen Strömungsbereich angeschlossen sind, der nicht mit Druckluft beaufschlagt wird.

Vorzugsweise ist die Druckluftquelle von der Flüssigkeitsversorgung räumlich beabstandet angeordnet, so dass die in einem Trockenbereich angeordnete Druckluftquelle mit allfälligen Ventilen zum Steuern und Unterteilen des Druckluftstroms in mehrere Strömungsbereiche des Druckluftstroms nicht mit dem Reinigungswasser des Nassbereichs in Berührung kommt. Dadurch können bei diesen Bauteilen Standardmaterialien zum Einsatz kommen, da das Reinigungswasser keine Weichmacher oder ähnliches aus den verwendeten Materialen herauslösen kann, die mit dem Endoskop in Berührung kommen könnten. Ausserdem eine solche Vorrichtung weniger wartungsintensiv, da die Bauteile im Trockenbereich weniger korrosionsgefährdet sind. Eine solche Beabstandung kann beispielsweise bewerkstelligt werden, in dem die Flüssigkeitsleitung und die Druckluftleitung jeweils in mehrere Flüssigkeitsverteilleitungen sowie in mehrere Druckluftverteilleitungen unterteilt werden, die jeweils dann miteinander paarweise verbunden werden und so in eine gemeinsame Zufuhrverteilleitung münden. Erst ab diesem Bereich muss ein Reinigungsmittel verträgliches Material für sämtliche Gerätekomponenten verwendet werden.

In einer anderen erfindungsgemässen Vorrichtung ist die Messvorrichtung ein Zeitmessgerät. Diese Vorrichtung enthält einen Behälter mit einem vorzugsweise integrierten Drucksensor, der mit Druckluft befüllbar ist und dazu dient, eine vorbestimmte Druckluftmenge durch die zu überprüfenden Endoskopkanäle zu leiten. Das Zeitmessgerät misst die Zeitdauer, die benötigt wird, um den Behälter zu leeren und damit die Zeit, die benötigt wird, um sämtliche Druckluft aus dem Behälter durch die Endoskopkanäle zu pressen. Der Zustand, ob der Behälter voll oder leer ist, wird von dem Luftdrucksensor des Behälters erfasst und an die Steuereinheit mit dem Zeitmessgerät gemeldet. Vorzugsweise ist in Strömungsrichtung hinter dem Behälter ein Rückschlagventil angeordnet, um sicherzustellen, dass der Drucksensor nicht durch zurückströmende Druckluft negativ beeinflusst wird. Vorzugsweise ist der Behälter fluidleitend mit einer vorzugsweise über ein Ventil steuerbare Druckluftquelle verbunden. Ein solches Ventil könnte ein pneumatisches Ventil sein, da dies kostengünstiger ist als ein hydraulisches Ventil.

Die Erfindung wird nachfolgend an mehreren Ausführungsbeispielen anhand der Zeichnungen näher erläutert.
Fig. 1 zeigt eine schematische Übersicht über die Anordnung einer Vorrichtung zur Adapteranschlussüberprüfung und zur Durchgängigkeitsüberprüfung von Endoskopkanälen in einer Endoskopaufbereitungsmaschine;
Fig. 2a zeigt ein Prinzipschaltbild einer Vorrichtung zur Adapteranschlussüberprüfung und zur Durchgängigkeitsüberprüfung von Endoskopkanälen;
Figur 2b und 2c zeigen eine beispielshafte Auswertung eines nichterfindungsgemässen Verfahrens mit einer Vorrichtung gemäss Figur 2a;
Fig. 3 zeigt ein Prinzipschaltbild einer erfindungsgemäßen Vorrichtung zur Adapteranschlussüberprüfung und zur Durchgängigkeitsüberprüfung von Endoskopkanälen;
Fig. 4 zeigt ein Prinzipschaltbild einer weiteren nicht-erfindungsgemäßen Vorrichtung zur Adapteranschlussüberprüfung und zur Durchgängigkeitsüberprüfung von Endoskopkanälen;
Fig. 5 zeigt ein Prinzipschaltbild einer weiteren nicht-erfindungsgemäßen Vorrichtung zur Adapteranschlussüberprüfung und zur Durchgängigkeitsüberprüfung von Endoskopkanälen.

Gleiche Bezugszeichen in den einzelnen Figuren bezeichnen gleiche beziehungsweise funktionell einander entsprechende Teile.

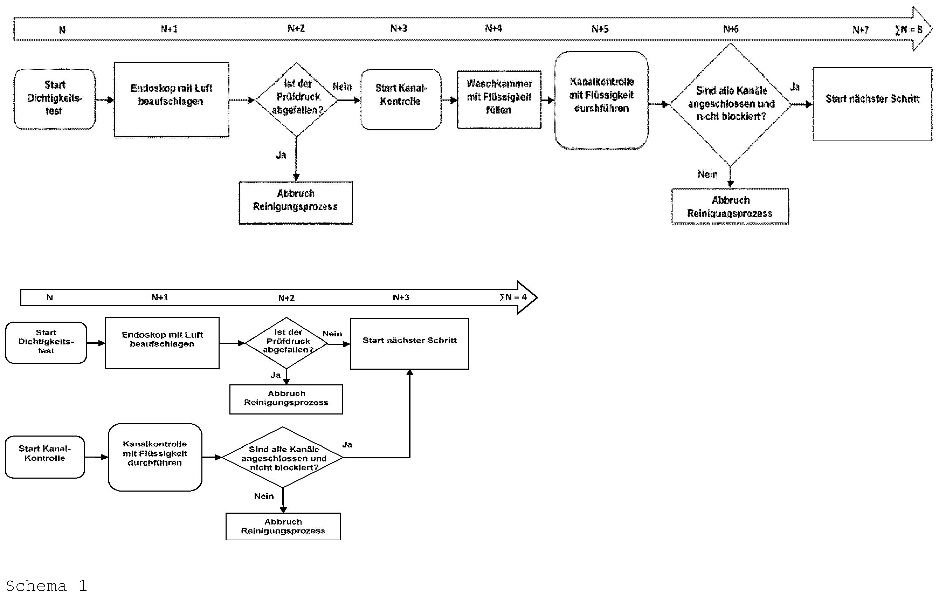

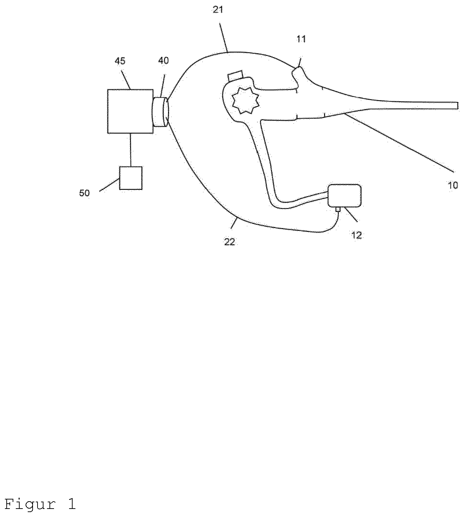

Figur 1 zeigt einen schematischen Überblick wie die Vorrichtung zur Adapteranschlussüberprüfung und zur Durchgängigkeitsüberprüfung von Endoskopkanälen mit einem Endoskop verbunden und in der Endoskopaufbereitungsmaschine angeordnet wird. Das Endoskop 10 weist eine Vielzahl von Endoskopkanälen auf, von denen hier zwei beispielhaft mit den Ziffern 11 und 12 bezeichnet sind. An diese Endoskopkanäle können Schläuche 21, 22 angeschlossen werden. Diese Schläuche 21, 22 sind Teil eines Adapters 40, der eine Vielzahl von Adapteranschlüssen aufweist, die vorzugsweise über eine Schnellkopplung an die erfindungsgemässe Kanalüberprüfungsvorrichtung 45 anschliessbar sind. Die Kanalüberprüfungsvorrichtung 45 ist mit einer Systemsteuerung 50 verbunden.

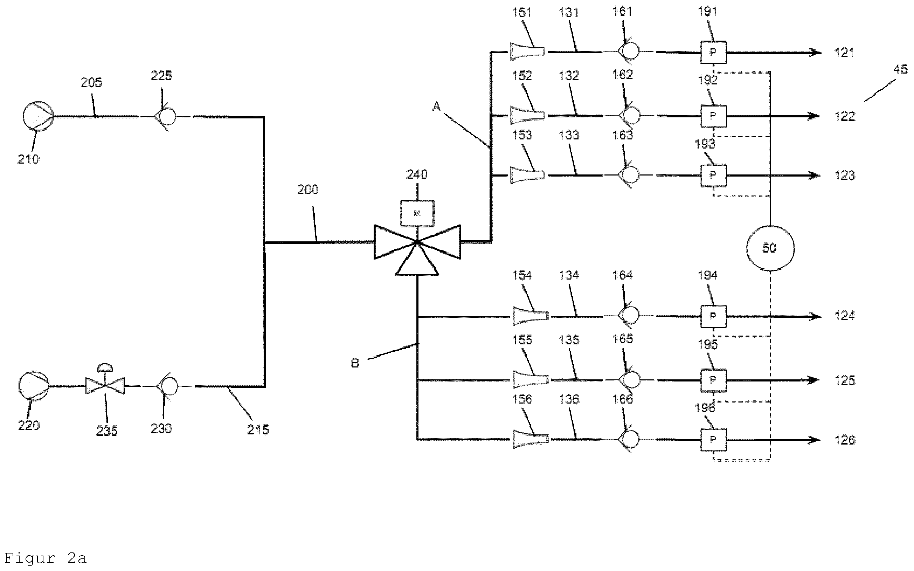

In Figur 2a wird eine erste Ausführungsform einer nichterfindungsgemässen Vorrichtung 45 gezeigt. Sie enthält eine Zufuhrleitung 200, die über eine Flüssigkeitsleitung 205 mit der Flüssigkeitsversorgung 210 verbunden ist, über welche Reinigungs- oder Spülflüssigkeit zugeführt werden kann. Die Flüssigkeitsversorgung 210 kann beispielsweise eine Pumpe beinhalten. Weiter ist die Zufuhrleitung 200 über eine Druckluftleitung 215 mit einer Druckluftquelle 220 verbunden, die beispielsweise ein elektrisch angetriebener Kompressor beinhalten kann. Das heisst, die Flüssigkeitsleitung 205 und die Druckluftleitung 215 münden in eine gemeinsame Zufuhrleitung 200. Die Zufuhrfuhrleitung 200 ist über einzelne Verteilleitungen 131, 132, 133, 134, 135, 136 mit einzelnen Adapteranschlüssen 121, 122, 123, 124, 125 und 126 über den Adapter mit den einzelnen Endoskopkanälen fluidleitend verbindbar.

In Strömungsrichtung ist nach der Flüssigkeitsversorgung 210 und nach der Druckluftquelle 220 jeweils vorzugsweise ein Rückschlagventil 225, 230 angeordnet. Das Rückschlagventil 225 ist so geschaltet, dass unter Druck stehende Flüssigkeit von der Flüssigkeitsleitung 205 zu den jeweiligen Adapteranschlüssen 121 bis 126 gelangen kann. Ausserdem schützt es die Flüssigkeitsversorgung 210 vor allfällig zurückfliessender Reinigungsflüssigkeit und vor Druckfluktuationen. Ebenso ist das Rückschlagventil 230 so geschaltet, dass Druckluft von der Druckluftquelle 220 zur Zufuhrleitung 200 und damit ebenfalls zu den Adapteranschlüssen 121 bis 126 gelangen kann. Ein weiteres Ventil 235 ist zwischen dem Rückschlagventil 230 und der Druckluftquelle 220 angeordnet und dient dazu, die Druckluft zu steuern. Das weitere Ventil 235 ist vorzugsweise ein pneumatisches Ventil.

In jeder einzelnen Verteilleitung 131, 132, 133, 134, 135, 136 ist je ein Drucksensor 191, 192, 193, 194, 195, 196 angeordnet, mit dem der Kanaldruck des über einen Adapter angeschlossenen Endoskopkanals gemessen wird. Dieser Messwert wird einer Steuereinheit 50 übermittelt, mit einem Referenzdruck dieses Endoskopkanals verglichen und ausgewertet. Die Steuereinheit 50 ist mit sämtlichen Drucksensoren 191, 192, 193, 194, 195, 196 verbunden.

Vorzugsweise enthält die erfindungsgemässe Vorrichtung ein hydraulisches Wegventil 240, mit dem der Druckluftstrom in mehrere Strömungsrichtungen lenkbar ist, und damit die individuelle Ansteuerung von mehreren Strömungsbereichen ermöglicht. Vorzugsweise ist ein solches hydraulisches Wegventil 240 ein hydraulisches Dreiwegventil. Je nach Stellung des Dreiwegventils wird der Druckluftzulaufstrom vollständig in einen ersten Strömungsbereich A oder vollständig in einen zweiten Strömungsbereich B gelenkt, oder der Druckluftzulaufstrom wird zwischen dem ersten und dem zweiten Strömungsbereich aufgeteilt.

Um sicherzustellen, dass sämtliche Endoskopkanäle mit genügend Druckluft beaufschlagt werden, können Düsen 151, 152, 153, 154, 155, 156 mit einem grösseren Einlassdurchmesser und einem kleineren Auslassdurchmesser die Druckluft kanalisieren. Alternativ können auch kostengünstigere, in einer Platte angeordnete Löcher verwendet werden. Dazu wird für jede Verteilleitung 131, 132, 133, 134, 135, 136 genau ein Loch in der Platte angebracht.

Vorzugsweise ist strömungsaufwärts vor jedem Drucksensor ein Rückschlagventil 161, 162, 163, 164, 165, 166 angeordnet, um ein Übersprechen der anderen Endoskopkanäle zu verhindern.

Figur 2b und 2c zeigen eine beispielshafte Auswertung eines nichterfindungsgemässen Verfahrens, bei dem jeder Endoskopkanal an eine Verteilleitung mit einem Drucksensor gemäss Figur 2a angeschlossen ist. Der Prüfzyklus beinhaltet jeweils drei Messungen. In einer ersten Messung wird nur der Strömungsbereich A mit Druckluft durchströmt, in einer zweiten Messung nur Strömungsbereich B und in einer dritten Messung beide Strömungsbereiche A und B.

In Figur 2b ist beispielsweise erkennbar, dass in sämtlichen drei Messungen der Kanaldruck von Kanal 1 abfällt. Zusätzlich fällt insbesondere bei Messung 1 der Kanaldruck von Kanal 4, der mit Kanal 1 verbunden ist, während bei Messung 2, der Kanaldruck von Kanal 4 nur unwesentlich vom Normwert abweicht. Damit ist klar, dass Kanal 1 nicht korrekt an die Endoskopmaschine angeschlossen wurde.

In Figur 2c ist beispielsweise erkennbar, dass in der ersten und dritten Messung der Kanaldruck von Kanal 1 stark erhöht ist, während er bei der zweiten Messung stark abfällt. Bei der ersten und der dritten Messung sind zusätzlich die im gleichen Strömungsbereich liegenden Kanaldrücke von Kanal 2 und Kanal 3 erhöht, während diese bei der zweiten Messung nicht vom Referenzdruck abweichen. Zusätzlich fällt insbesondere bei der Messung 1 der Kanaldruck von Kanal 4, der mit Kanal 1 verbunden ist, während bei Messung 2, der Kanaldruck von Kanal 4 nur unwesentlich vom Normwert abweicht. Damit ist klar, dass Kanal 1 verstopft ist.

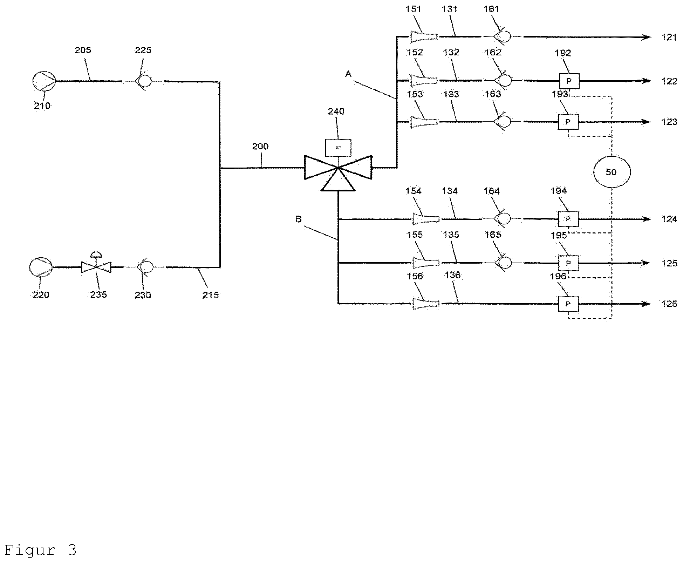

Figur 3 zeigt eine Ausführungsform der erfindungsgemässen Vorrichtung, die im Wesentlichen der Ausführungsform gemäss Figur 2a entspricht. Im Gegensatz zu der in Figur 2a gezeigten Ausführungsform weist im ersten Strömungsbereich A die Verteilleitung 131 keinen Drucksensor 191 auf. Ausserdem weist im zweiten Strömungsbereich B die Verteilleitung 136 kein Rückschlagventil 166 auf. Es sind auch Ausführungsformen möglich, bei denen nur der Drucksensor in der Verteilleitung 131 oder nur das Rückschlagventil in der Verteilleitung 136 fehlt.

In einem ersten Schritt des erfindungsgemässen Verfahrens wird die Druckluft vollständig in den ersten Strömungsbereich A geleitet, der mit drei Endoskopkanälen 1, 2 und 3 verbunden ist (nicht gezeigt), wobei wegen des fehlenden Drucksensors in der Verteilerleitung 131 kein Druck gemessen wird. Für die ebenfalls nicht gezeigten Endoskopkanäle 2 und 3, die mit den Verteilerleitungen 132 und 133 verbunden sind, wird jeweils der Kanaldruck von jedem einzelnen Endoskopkanal mit den entsprechenden Drucksensoren 192 und 193 gemessen und der Steuereinheit 50 übermittelt. Ausserdem werden die mit den ebenfalls nicht gezeigten Endoskopkanälen 4, 5 und 6 verbunden Verteilerleitungen 134, 135 und 136, der Kanaldruck mit den entsprechenden Drucksensoren 194, 195 und 196 gemessen und der Steuereinheit übermittelt.

In einem zweiten Schritt wird die Druckluft vollständig in den zweiten Strömungsbereich B geleitet, der hier beispielhaft mit drei weiteren Endoskopkanälen 4, 5, 6 verbunden ist (nicht gezeigt), wobei jeweils der Kanaldruck von jedem einzelnen Endoskopkanal mittels eines Drucksensors 192, 193, 194, 195, 196 gemessen und der Steuereinheit 50 übermittelt wird.

In einem optionalen dritten Schritt wird die Druckluft in den ersten Strömungsbereich A und den zweiten Strömungsbereich B geleitet und für die Endoskopkanäle 2, 3, 4, 5 und 6 (nicht gezeigt) jeweils der Kanaldruck mit dem Drucksensor 192, 193, 194, 195 und 196 gemessen und der Steuereinheit 50 übermittelt.

Sämtliche gemessenen Kanaldrücke werden in der Steuereinheit 50 mit den entsprechenden Referenzdrücken verglichen. Da der Endoskopkanal 1 mit einem weiteren Endoskopkanal verbunden ist, kann auch für diesen ermittelt werden, ob er frei und/oder korrekt angeschlossen ist.

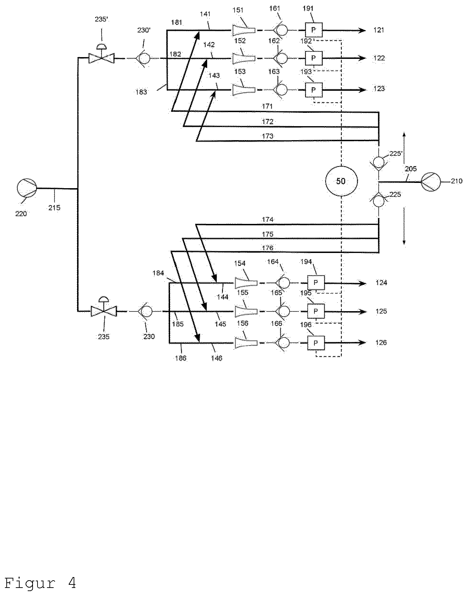

Figur 4 zeigt eine weitere Ausführungsform einer nichterfindungsgemässen Vorrichtung, die im Wesentlichen der Ausführungsform gemäss Figur 2a entspricht. Im Gegensatz zu der in Figur 2a gezeigten Ausführungsform wird die Zufuhrleitung nicht direkt mit der Flüssigkeitsleitung 205 und der Druckluftleitung 215 verbunden, sondern diese werden erst in Flüssigkeitsverteilleitungen 171, 172, 173, 174, 175, 176 sowie in Druckluftverteilleitungen 181, 182, 183, 184, 185, 186 unterteilt, die jeweils dann miteinander paarweise verbunden werden und so die Zufuhrverteilleitungen 141, 142, 143, 144, 145, 146 bilden. Diese Anordnung erlaubt eine räumliche Beabstandung von Druckluftquelle 220 und Flüssigkeitsversorgung 210. Dadurch kann sichergestellt werden, dass beispielsweise die beiden weiteren Ventile 235, 235' und die beiden Rückschlagventile 230, 230' nicht in Berührung mit der Reinigungsflüssigkeit kommen. Dadurch können diese aus Standardwerkstoffen gefertigt sein, da beispielsweise nicht sichergestellt werden muss, dass diese keine Weichmacher verlieren und auch weniger korrosionsgefährdet sind.

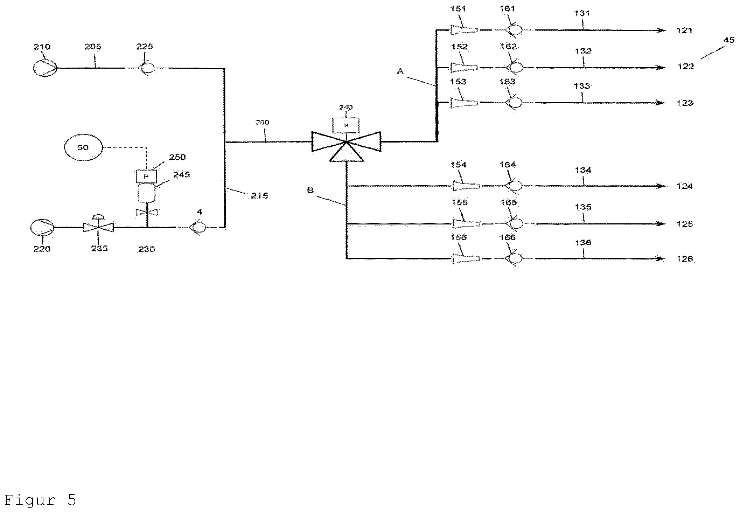

Figur 5 zeigt eine weitere Ausführungsform einer nichterfindungsgemässen Vorrichtung, die von der Ausführungsform gemäss Figur 2a dahingehend abweicht, dass die einzelnen Verteilleitungen 131, 132, 133, 134, 135, 136 keine Drucksensoren aufweisen, aber zwischen dem weiteren Ventil 235 und dem Rückschlagventil 230 ein Behälter 245 mit einem vorzugsweise integrierten Luftdrucksensor 250 angeordnet ist, der mit einer Steuereinheit 50 verbunden ist, die eine Zeitmessvorrichtung enthält. Dieser Behälter 245 ist dazu bestimmt, mit Druckluft gefüllt zu werden und Zustand «Behälter voll» wird von dem Drucksensor 250 erfasst und der Steuereinheit gemeldet. Diese Druckluft wird durch die angeschlossenen Endoskopkanäle gepresst. Ist alle Druckluft aus dem Behälter durch die Endoskopkanäle gepresst, so wird der Zustand "Behälter leer" von dem Luftdrucksensor erfasst und an die Steuereinheit gemeldet. Die Steuereinheit 50 misst mit einer Zeitmessvorrichtung die Zeitdauer, die benötigt wird, um sämtliche Druckluft aus dem Behälter durch die Endoskopkanäle zu pressen. Bei freien Endoskopkanälen, bekanntem Querschnitt der Endoskopkanäle, bekanntem Volumen des Behälters und bekanntem, konstantem Druck der Druckluft muss der Behälter innerhalb einer vorgegebenen Zeitdauer vollständig entleert sein. Ist dies nicht der Fall, ist ein Endoskopkanal ganz oder teilweise blockiert.

## Patentansprüche

1. Verfahren zur gleichzeitigen Adapteranschlussüberprüfung und Durchgängigkeitsüberprüfung von Endoskopkanälen in einer Endoskopaufbereitungsmaschine, wobei diese Überprüfung mit Gas erfolgt und das Gas über ein Wegventil in einzelne oder mehrere Strömungsbereiche geleitet wird, wobei n Endoskopkanäle an einem ersten Strömungsbereich A und m Endoskopkanäle an einem zweiten Strömungsbereich B angeschlossen sind, wobei ein erster Endoskopkanal, der an den Strömungsbereich A angeschlossen ist, mit einem an dem Strömungsbereich B angeschlossenen Endoskopkanal verbunden ist, wobei
i. die Druckluft vollständig in den ersten Strömungsbereich geleitet wird, der mit n Endoskopkanälen des ersten Strömungsbereichs verbunden ist, wobei für den ersten Endoskopkanal der Kanaldruck nicht gemessen wird und jeweils der Kanaldruck für die n-1 Endoskopkanäle des ersten Strömungsbereichs und m Endoskopkanäle des zweiten Strömungsbereichs mittels eines Drucksensors gemessen und einer Steuereinheit übermittelt wird,
ii. die Druckluft vollständig in einen zweiten Strömungsbereich geleitet wird, der mit m Endoskopkanälen des zweiten Strömungsbereichs verbunden ist, wobei für sämtliche n-1 Endoskopkanäle des ersten Strömungsbereichs und m Endoskopkanäle des zweiten Strömungsbereichs jeweils der Kanaldruck mittels eines Drucksensors gemessen und einer Steuereinheit übermittelt wird,
iii. optional die Druckluft in den ersten und den zweiten Strömungsbereich geleitet wird, und für die n-1 Endoskopkanäle des ersten Strömungsbereichs und die m Endoskopkanäle des zweiten Strömungsbereichs jeweils der Kanaldruck mittels eines Drucksensors gemessen und der Steuereinheit übermittelt wird, und
iv. in der Steuereinheit die gemessenen Kanaldrücke mit Referenzdrücken verglichen und ausgewertet werden.

2. Verfahren gemäss Anspruch 1, **dadurch gekennzeichnet, dass** die Adapteranschlussüberprüfung und die Durchgängigkeitsüberprüfung von Endoskopkanälen gleichzeitig zur Überprüfung der Dichtigkeit des Endoskops durchgeführt wird.

3. Verfahren gemäss Anspruch 1 oder 2, **dadurch gekennzeichnet, dass** während der Überprüfung gleichzeitig die Reinigungsmaschine mit der Reinigungsflüssigkeit befüllt wird.

4. Verfahren gemäss einem der vorangehenden Ansprüche, **dadurch gekennzeichnet, dass** das Gas über pneumatisches Wegventil, und vorzugsweise ein Dreiwegventil, in einzelne oder mehrere Strömungsbereiche geleitet wird.

5. Verfahren gemäss einem der vorangehenden Ansprüche, **dadurch gekennzeichnet, dass** der Kanaldruck von jedem einzelnen Endoskopkanal mittels eines Drucksensors gemessen, mit einem Referenzwert für diesen Endoskopkanal verglichen und ausgewertet wird.

6. Verfahren gemäss Anspruch 1, **dadurch gekennzeichnet, dass** es sich bei dem Gas zur Überprüfung um medizinische Druckluft handelt.

7. Verfahren gemäss einem der Ansprüche 1 bis 4, **dadurch gekennzeichnet, dass** eine vorbestimmte Druckluftmenge durch die zu überprüfenden Endoskopkanäle geleitet wird und die Zeitdauer hierfür gemessen und ausgewertet wird.

8. Vorrichtung eingerichtet
zur Durchführung des Verfahrens gemäss einem der Ansprüche 1 bis 7, enthaltend eine Druckluftquelle (220), die über eine Druckluftleitung (215) mittels eines Adapters (40) mit den Endoskopkanälen fluidleitend verbindbar ist, eine Flüssigkeitsversorgung (210), die über eine Flüssigkeitsleitung (205) mittels eines Adapters (40) mit den Endoskopkanälen fluidleitend verbindbar ist, wobei die Druckluftleitung (215) und die Flüssigkeitsleitung (205) in eine gemeinsame Zufuhrleitung (200) oder in gemeinsame Zufuhrverteilleitungen (141, 142, 143, 144, 145, 146) münden,
wenigstens eine Messeinrichtung (191, 192, 193, 194, 195, 196, 250) zum Messen eines auf die Durchlässigkeit der Endoskopkanäle bezogenen Messwertes, und
eine Steuerungseinheit zum Erfassen und Auswerten der Messwerte,
wobei die Messeinrichtung ein Drucksensor (191, 192, 193, 194, 195, 196) ist und
wobei die Vorrichtung ein Wegventil (240) aufweist, das dazu dient, den Druckluftstrom individuell in zwei oder mehrere verschiedene Strömungsbereiche zu leiten,
wobei ein erster Endoskopkanal, der an einem ersten Strömungsbereich A angeschlossen ist, mit einem an einem anderen Strömungsbereich B angeschlossenen Endoskopkanal verbunden ist,
wobei mit Ausnahme des ersten Endoskopkanals für jeden Endoskopkanal ein Drucksensor angeordnet ist.

9. Vorrichtung gemäss Anspruch 8, **dadurch gekennzeichnet, dass** das Wegventil (240) ein Dreiwegventil ist.

10. Vorrichtung gemäss einem der Ansprüche 8 bis 9, **dadurch gekennzeichnet, dass** strömungsaufwärts vor jedem Drucksensor ein Rückschlagventil angeordnet ist.

11. Vorrichtung gemäss einem der Ansprüche 8 oder 10, **dadurch gekennzeichnet, dass** mit Ausnahme eines Drucksensors strömungsaufwärts vor allen Drucksensoren (191, 192, 193, 194, 195, 196) ein Rückschlagventil (161, 162, 163, 164, 165) angeordnet ist.

12. Vorrichtung gemäss Anspruch 8 bis 10, **dadurch gekennzeichnet, dass** die Druckluftquelle (220) von der Flüssigkeitsversorgung (210) beabstandet angeordnet ist, so dass die in einem Trockenbereich angeordnete Druckluftquelle (220) mit allfälligen Ventilen (235, 235') zum Steuern des Druckluftstroms nicht mit dem Nassbereich in Berührung kommt.

13. Vorrichtung gemäss Anspruch 8, **dadurch gekennzeichnet, dass** die Messeinrichtung ein Zeitmessgerät ist.

## Claims

1. A method for simultaneous verification of adapter connection
and permeability of endoscope channels in an endoscope preparation machine, wherein this verification is carried out using gas and the gas is conducted via a directional control valve into individual or multiple flow areas, wherein n endoscope channels are connected to a first flow area A and m endoscope channels are connected to a second flow area B, wherein a first endoscope channel connected to the flow area A is connected to an endoscope channel connected to the flow area B, wherein
i. the compressed air is conducted completely into the first flow area, which is connected to n endoscope channels of the first flow area, wherein the channel pressure is not measured for the first endoscope channel and the channel pressure is measured for each of the n-1 endoscope channels of the first flow area and m endoscope channels of the second flow area by means of a pressure sensor and transmitted to a control unit,
ii. the compressed air is conducted completely into a second flow area, which is connected to m endoscope channels of the second flow area, wherein for all n-1 endoscope channels of the first flow area and m endoscope channels of the second flow area, the channel pressure is measured in each case by means of a pressure sensor and transmitted to a control unit,
iii. optionally the compressed air is conducted into the first and the second flow area, and for the n-1 endoscope channels of the first flow area and the m endoscope channels of the second flow area, the channel pressure is measured in each case by means of a pressure sensor and transmitted to the control unit, and
iv. in the control unit, the measured channel pressures are compared to reference pressures and evaluated.

2. The method according to Claim 1, **characterized in that** the verification of adapter connection and permeability of endoscope channels is carried out simultaneously with the verification of the tightness of the endoscope.

3. The method according to Claim 1 or 2, **characterized in that** the cleaning machine is filled with the cleaning liquid simultaneously during the verification.

4. The method according to any one of the preceding claims, **characterized in that** the gas is conducted via a pneumatic directional control valve, and preferably a three-way valve, into individual or multiple flow areas.

5. The method according to any one of the preceding claims, **characterized in that** the channel pressure of each individual endoscope channel is measured by means of a pressure sensor, compared to a reference value for this endoscope channel, and evaluated.

6. The method according to Claim 1, **characterized in that** the gas for the verification is medical compressed air.

7. The method according to any one of Claims 1 to 4, **characterized in that** a predetermined amount of compressed air is conducted through the endoscope channels to be verified and the duration of this is measured and evaluated.

8. A device configured to carry out the method according to any one of Claims 1 to 7, containing
a compressed air source (220) which is fluidically connectable to the endoscope channels via a compressed air line (215) by means of an adapter (40),
a liquid supply (210) which is fluidically connectable to the endoscope channels via a liquid line (205) by means of an adapter (40), wherein the compressed air line (215) and the liquid line (205) discharge into a common supply line (200) or into common supply distribution lines (141, 142, 143, 144, 145, 146),
at least one measuring device (191, 192, 193, 194, 195, 196, 250) for measuring a measured value related to the permeability of the endoscope channels, and
a control unit for recording and evaluating the measured values,
wherein the measuring device is a pressure sensor (191, 192, 193, 194, 195, 196) and
wherein the device has a directional control valve (240) for conducting the flow of compressed air individually into two or more different flow areas,
wherein a first endoscope channel connected to a first flow area A is connected to an endoscope channel connected to another flow area B,
wherein, with the exception of the first endoscope channel, a pressure sensor is arranged for each endoscope channel.

9. The device according to Claim 8, **characterized in that** the directional control valve (240) is a three-way valve.

10. The device according to any one of Claims 8 to 9, **characterized in that** a check valve is arranged upstream of each pressure sensor.

11. The device according to any one of Claims 8 or 10, **characterized in that**, with the exception of one pressure sensor, a check valve (161, 162, 163, 164, 165) is arranged upstream of all pressure sensors (191, 192, 193, 194, 195, 196) .

12. The device according to Claims 8 to 10, **characterized in that** the compressed air source (220) is arranged spaced apart from the liquid supply (210), so that the compressed air source (220) arranged in a dry area with any valves (235, 235') for controlling the compressed air flow does not come into contact with the wet area.

13. The device according to Claim 8, **characterized in that** the measuring device is a time measuring device.

## Revendications

1. Procédé de vérification simultanée de connexion d'un adaptateur et de l'ouverture des canaux de l'endoscope dans une machine de conditionnement d'endoscope, dans lequel cette vérification est effectuée avec du gaz et le gaz est dirigé dans une ou plusieurs zones d'écoulement via une vanne de commande directionnelle, dans lequel n canaux d'endoscope sont connectés à une première zone d'écoulement A et m canaux d'endoscope sont connectés à une seconde zone d'écoulement B, dans lequel un premier canal d'endoscope, qui est connecté à la zone d'écoulement A, est relié à un canal d'endoscope connecté à la zone d'écoulement B, dans lequel
i. l'air comprimé est dirigé complètement dans la première zone d'écoulement, qui est reliée à n canaux d'endoscope de la première zone d'écoulement, dans lequel la pression de canal n'est pas mesurée pour le premier canal d'endoscope et la pression de canal pour les n-1 canaux d'endoscope de la première zone d'écoulement et m canaux d'endoscope de la seconde zone d'écoulement est mesurée par un capteur de pression et transmise à une unité de commande,
ii. l'air comprimé est dirigé complètement dans une seconde zone d'écoulement, qui est reliée à m canaux d'endoscope de la seconde zone d'écoulement, dans lequel pour tous les n-1 canaux d'endoscope de la première zone d'écoulement et m canaux d'endoscope de la seconde zone d'écoulement, la pression de canal est mesurée par un capteur de pression et transmise à une unité de commande,
iii. éventuellement, l'air comprimé est dirigé dans la première et la seconde zone d'écoulement, et pour les n-1 canaux d'endoscope de la première zone d'écoulement et les m canaux d'endoscope de la seconde zone d'écoulement, la pression de canal est mesurée par un capteur de pression et transmise à l'unité de commande, et
iv. dans l'unité de commande, les pressions de canal mesurées sont comparées à des pressions de référence et évaluées.

2. Procédé selon la revendication 1, **caractérisé en ce que** la vérification de connexion de l'adaptateur et de l'ouverture des canaux de l'endoscope sont effectuées simultanément pour vérifier l'étanchéité de l'endoscope.

3. Procédé selon la revendication 1 ou 2, **caractérisé en ce que** la machine de nettoyage est remplie de liquide de nettoyage simultanément pendant la vérification.

4. Procédé selon l'une des revendications précédentes, **caractérisé en ce que** le gaz est dirigé via une soupape de commande directionnelle pneumatique, et de préférence une vanne à trois voies, dans une ou plusieurs zones d'écoulement.

5. Procédé selon l'une des revendications précédentes, **caractérisé en ce que** la pression de canal de chaque canal d'endoscope individuel est mesurée par un capteur de pression, comparée à une valeur de référence pour ce canal d'endoscope et évaluée.

6. Procédé selon la revendication 1, **caractérisé en ce que** le gaz à vérifier est de l'air comprimé médical.

7. Procédé selon l'une des revendications 1 à 4, **caractérisé en ce qu'**une quantité prédéterminée d'air comprimé est dirigée à travers les canaux d'endoscope à vérifier et la durée de cette opération est mesurée et évaluée.

8. Dispositif mis en place pour la mise en œuvre du procédé selon l'une des revendications 1 à 7, contenant une source d'air comprimé (220) qui peut être reliée fluidiquement aux canaux d'endoscope via une conduite d'air comprimé (215) au moyen d'un adaptateur (40),
une alimentation en liquide (210) qui peut être reliée fluidiquement aux canaux de l'endoscope via une conduite de liquide (205) au moyen d'un adaptateur (40), dans lequel la conduite d'air comprimé (215) et la conduite de liquide (205) débouchent dans une conduite d'alimentation commune (200) ou dans des conduites de distribution d'alimentation communes (141, 142, 143, 144, 145, 146),
au moins un dispositif de mesure (191, 192, 193, 194, 195, 196, 250) pour mesurer une valeur de mesure liée à l'ouverture des canaux d'endoscope, et
une unité de commande pour enregistrer et évaluer les valeurs de mesure,
dans lequel le dispositif de mesure est un capteur de pression (191, 192, 193, 194, 195, 196) et
dans lequel le dispositif présente une vanne de commande directionnelle (240) qui sert à diriger le flux d'air comprimé individuellement dans deux zones d'écoulement différentes ou plus,
dans lequel un premier canal d'endoscope connecté à une première zone d'écoulement A est relié à un canal d'endoscope connecté à une autre zone d'écoulement B,
dans lequel, à l'exception du premier canal d'endoscope, un capteur de pression est disposé pour chaque canal d'endoscope.

9. Dispositif selon la revendication 8, **caractérisé en ce que** la vanne de commande directionnelle (240) est une vanne à trois voies.

10. Dispositif selon l'une des revendications 8 à 9, **caractérisé en ce qu'**un clapet anti-retour est disposé en amont de chaque capteur de pression.

11. Dispositif selon l'une des revendications 8 ou 10, **caractérisé en ce qu'**à l'exception d'un capteur de pression, un clapet anti-retour (161, 162, 163, 164, 165) est disposé en amont de tous les capteurs de pression (191, 192, 193, 194, 195, 196).

12. Dispositif selon les revendications 8 à 10, **caractérisé en ce que** la source d'air comprimé (220) est disposée à distance de l'alimentation en liquide (210), de sorte que la source d'air comprimé (220) disposée dans une zone sèche avec toute les soupapes (235, 235') de commande d'écoulement de l'air comprimé n'entrent pas en contact avec la zone humide.

13. Dispositif selon la revendication 8, **caractérisé en ce que** le dispositif de mesure est un dispositif de mesure du temps.
